# EUROPÄISCHE PATENTSCHRIFT

(11) **EP 1 030 923 B1**
(45) Veröffentlichungstag und Bekanntmachung des Hinweises auf die Patenterteilung: **09.02.2005**
(21) Anmeldenummer: 98965067.6
(22) Anmeldetag: 13.11.1998
(51) Int. Cl.: C12N 15/37, C12N 15/81, C12N 7/04, C07K 14/025

(54) **VERFAHREN ZUR GEWINNUNG VON VIRUSÄHNLICHEN PARTIKELN AUF DER BASIS VON POLYOMAVIREN**
METHOD FOR PRODUCING VIRUS-LIKE PARTICLES ON THE BASIS OF POLYOMA VIRUSES
PROCEDE D'OBTENTION DE PARTICULES SEMBLABLES A DES VIRUS A BASE DE VIRUS DE POLYOME

(30) Priorität: 13.11.1997 DE 19750220
(43) Veröffentlichungstag der Anmeldung: 30.08.2000
(73) Patentinhaber: MAX-DELBRÜCK-CENTRUM FÜR MOLEKULARE MEDIZIN, 13125 Berlin (DE)
(72) Erfinder: SCHERNECK, Siegfried, D-10365 Berlin (DE); SASNAUSKAS, Kestutis, LT-2050 Vilnius (LT); ULRICH, Rainer, D-10245 Berlin (DE)
(74) Vertreter: Baumbach, Friedrich, Dr.
(86) Internationale Anmeldenummer: PCT/DE1998/003329
(87) Internationale Veröffentlichungsnummer: WO 1999/025837

(56) Entgegenhaltungen:
- FORSTOVA J ET AL: "POLYOMA VIRUS PSEUDOCAPSIDS AS EFFICIENT CARRIERS OF HETEROLOGOUS DNA INTO MAMMALIAN CELLS" HUMAN GENE THERAPY, Bd. 6, Nr. 3, März 1995, Seiten 297-306, XP000645479
- E.T. GILLOCK ET AL.: "Polyomavirus major capsid protein VP1 is capable of packaging cellular DNA when expressed in the baculovirus system" J. VIROLOGY, Bd. 71, Nr. 4, April 1997, Seiten 2857-2865, XP002100672 AM.SOC.MICROBIOL.,WASHINGTON,US
- L. MONTROSS ET AL.: "Nuclear assembly of polyomavirus capsids in insect cells expressing the major capsid protein VP1" J. VIROLOGY, Bd. 65, Nr. 9, September 1991, Seiten 4991-4998, XP002100673 AM.SOC.MICROBIOL.,WASHINGTON,US in der Anmeldung erwähnt
- D.M. SALUNKE ET AL.: "Polymorphism in the assembly of polyomavirus capsid protein VP1" BIOPHYSICAL JOURNAL, Bd. 56, November 1989, Seiten 887-900, XP002100674
- D.M. SALUNKE ET AL.: "Self-assembly of purified polyomavirus capsid protein VP1" CELL, Bd. 46, 12. September 1986, Seiten 895-904, XP002100675 CELL PRESS,CAMBRIDGE,MA,US; in der Anmeldung erwähnt
- Y. SHISHIDO ET AL.: "Assembly of JC virus-like particles in COS7 cells" J. MEDICAL VIROLOGY, Bd. 51, Nr. 4, April 1997, Seiten 265-272, XP002100676 Wiley-Liss, Inc. NY, US
- D. CHANG ET AL.: "Self-assembl of the JC virus major capsid protein, VP1, expressed in insect cells" J. GENERAL VIROLOGY, Bd. 78, Nr. 6, Juni 1997, Seiten 1435-1439, XP002100677 READING, BERKS, GB in der Anmeldung erwähnt
- K.U. JANSEN ET AL.: "Vaccination with yeast-expressed cottentail rabbit papillomyvirus (CRPV) virus-like particles protects rabbits from CRPV-induced papiloma formation" VACCINE, Bd. 13, Nr. 16, 1995, Seiten 1509-1514, XP002100678 BUTTERWORTH-HEINEMANN LTD, BOSTON, MA, USA
- M.P. NEEPER ET AL.: "Expression of the major capsid protein of human papillomavirus type 11 in Saccharomyces cerevisiae" GENE, Bd. 180, 1996, Seiten 1-6, XP002100679 ELSEVIER SCIENCE PUBLISHERS,B.V.,AMSTERDAM,NL;
- SASAGAWA T ET AL: "SYNTHESIS AND ASSEMBLY OF VIRUS-LIKE PARTICLES OF HUMAN PAPILLOMAVIRUSES TYPE 6 AND TYPE 16 IN FISSION YEAST SCHIZOSACCHAROMYCES POMBE" VIROLOGY, Bd. 206, Januar 1995, Seiten 126-135, XP000615231
- K.J. HOFMANN ET AL.: "Sequence dertermination of human papillomavirus type 6a and assembly of virus like particles in Saccharomyces cerevisiae" VIROLOGY, Bd. 209, 1995, Seiten 506-518, XP002100680 ACADEMIC PRESS, INC.,NEW YORK, US
- R.B. MORELAND AND R.L. GARCEA: "Characterization of a nuclear localization sequence in the polyomavirus capsid protein VP1" VIROLOGY, Bd. 185, 1991, Seiten 513-518, XP002100681 ACADEMIC PRESS, INC.,NEW YORK, US
- R. ULRICH ET AL.: "Hamster Polyomavirus-encoded proteins: gene cloning, heterologous expression and imunoreactivity" VIRUS GENES, Bd. 12, Nr. 3, 1996, Seiten 265-274, XP002100682 Kluwer Academic Publishers, Boston, Manufactured in The Netherlands
- K. SASNAUSKAS ET AL.: "Cloning and analysis of a Candida maltosa gene which confers resistance to formaldehyde in Saccharomyces cerevisiae" GENE, Bd. 122, 1992, Seiten 207-211, XP002100683 ELSEVIER SCIENCE PUBLISHERS,B.V.,AMSTERDAM,NL;
- DATABASE SCISEARCH ELSEVIER SCIENCE PUBLISHERS, AMSTERDAM, NL SASNAUSKAS K ET AL: "Yeast cells allow high-level expression and formation of polyomavirus-like particles" XP002100684 & BIOLOGICAL CHEMISTRY, (MAR 1999) VOL. 380, NO. 3, PP. 381-386. PUBLISHER: WALTER DE GRUYTER & CO, GENTHINER STRASSE 13, D-10785 BERLIN, GERMANY. ISSN: 1431-6730.,

## Beschreibung

Die Erfindung betrifft ein Verfahren zur Gewinnung von virusähnlichen Partikeln auf der Basis von Polyomaviren. Anwendungsgebiete sind die Medizin, u. a. Gentherapie, Vakzineentwicklung und Diagnostik, sowie die Veterinärmedizin.

Die somatische Gentherapie hat in den vergangegenen Jahren eine rasante Entwicklung genommen. Inzwischen werden erste klinische Studien durchgeführt. Dennoch ist die Applikationsform des 'heilenden Gens' nach wie vor mit Problemen behaftet. Obwohl virale Vektoren (z.B. auf der Basis von Retroviren oder Adenoviren) eine Reihe von Vorteilen bieten, existieren auch eine Reihe von Sicherheitsproblemen, z. B. ein onkogenes Potential solcher Vektoren und deren Infektiosität, verursacht z. B. durch Reversion deletierter viraler Vektoren zum virulenten Wildtyp in packaging-Zellinien (Übersichten in J.-M.H. Vos, Herausgeber, Viruses in human gene therapy, Carolina Academic press, 1995; Schofield und Caskey, 1995, British Med. Bull. 51, 56-71). Gegenüber solchen Virusvektoren besitzen virus-ähnliche Partikel (virus-like particles: VLPs) den Vorteil, daß sie nicht infektiös sind und kein tumorigenes Potential besitzen. Solche VLPs können durch Expression von viralen Kapsid- und Hüllproteinen in verschiedenen heterologen Expressionssystemen erzeugt werden (Übersicht in Ulrich et al., Adv. Virus Res. 50, 1997). Besondere Aufmerksamkeit für den Gentransfer erlangten virus-ähnliche Partikel auf der Basis des Hauptkapsidproteins VP1 ('Virusprotein 1') von Polyomaviren. Bisher sind VP1 verschiedener Polyomaviren in Insektenzellen exprimiert worden. Das rekombinante VP1 von nicht-humanen und humanen Polyomaviren lagerte sich in den Insektenzellen spontan zu VLPs zusammen (Montross et al., 1991, J. Virol. 65, 4991-4998; Chang et al., 1997, J. Gen. Virol. 78, 1435-1439). Bei *E*. *coli*-Expression von Polyomavirus-VP1 ist es gelungen, Kapsomere zu exprimieren, die *in vitro* zu VLPs re-assoziiert werden können (Salunke et al., 1986, Cell 46, 895-904).

Die Verwendung solcher VP1-Partikel im medizinischen Bereich erfordert jedoch eine weitergehende Reinigung, um eventuell vorhandene Fremdstoffe (z. B. toxisch wirkende Verunreinigungen) zu beseitigen.

Ziel der Erfindung ist die Entwicklung eines Verfahrens zur Gewinnung von virusähnlichen Partikeln für Gentherapie, Diagnostik und Vakzineentwicklung. Dieses Verfahren soll die hocheffiziente Produktion von nicht-infektiösen, Endotoxinfreien virusähnlichen Partikeln ermöglichen, die für humane Anwendungen geeignet sind.

Die Erfindung wird gemäß dem Anspruch 1 realisiert, die Unteransprüche sind Vorzugsvarianten.

Der wesentliche Bestandteil des erfindungsgemäßen Verfahrens ist ein Hefe-Expressionssystem, das die Hochexpression von Kapsid-ähnlichen Partikeln von Polyomaviren erlaubt. Diese Partikel können in großer Menge aus Hefezellen isoliert und durch einfache Zentrifugationsschritte gereinigt werden.

Das Verfahren beinhaltet die PCR-Amplifikation und Klonierung einer Polyomavirus-VP1-kodierenden Sequenz. Diese DNA-Sequenz wird in einen Hefeexpressionsvektor mit einer Hefe-spezifischen Expressionseinheit (Promoter und Transkriptionsstopsignal) eingebaut. Die Expression des VP1 erfolgt in Hefezellen, bevorzugt im *Saccharomyces cerevisiae* Stamm AH 22 (leu2 his4). Nach Aufschluß der Hefezellen mit Hilfe von Glasperlen werden die virusähnlichen Partikel durch Ultrazentrifugation (Saccharose-Kissen) sedimentiert. Durch anschließende Ultrazentrifugation im Cäsiumchloridgradienten werden die Partikel gereinigt. Die anschließende Analyse der Partikel erfolgt durch Elektronenmikroskopie.

Durch die Erfindung wird ein neuartiges Expressionssystem für virusähnliche Partikel von Polyomaviren verfügbar, das gegenüber den bisher verfügbaren Systemen in *E. coli* bzw. Insektenzellen wesentliche Vorteile beinhaltet. So erlauben Hefe-Expressionssysteme die biotechnologische Produktion von Antigenen. Desweiteren besitzen Hefen keine Toxine, die unerwünschte Nebenwirkungen bei human- oder veterinärmedizinischen Anwendungen hervorrufen würden. Ein weiterer Vorteil ist, daß Hefesysteme bereits für die humane Vakzineproduktion zugelassen sind: Die rekombinante HBV-Vakzine basiert auf Hefe-exprimiertem HBsAg.

Es ist besonders überraschend, daß die Hefezellen das Polyomavirus-VP1 in großer Menge synthetisieren. Außerdem können die VP1-Partikel nach der Lyse der Hefezellen in großer Menge mit einfachen Methoden isoliert und gereinigt werden. Sie sind u. a. verwendbar zum Nachweis von Infektionen mit humanen Polyomaviren, als Vehikel für den Gentransfer, für eine Vakzineentwicklung gegen humane Polyomaviren oder gegen andere Viren (z. B. durch Präsentation von Fremdepitopen auf HaPV-VP1-Kapsiden).

Die Erfindung soll nachfolgend durch ein Ausführungsbeispiel näher erläutert werden:

### Ausführungsbeispiel

### 1. Gewinnung der HaPV-VP1 kodierenden Sequenz

HaPV-Virionen werden aus Epitheliomen HaPV-infizierter Z3 Hamster isoliert und durch Saccharose/Cäsiumchlorid-Gradientenzentrifugation gereinigt. Die Virionen werden durch Zugabe von Probenpuffer denaturiert, im SDS-Polyacrylamid-Gel aufgetrennt und anschließend auf eine PVDF-Membran transferiert. Die VP1-Proteinbande wird ausgeschnitten und mit Hilfe der Edman-Degradation am N-Terminus sequenziert. Entsprechend der N-terminalen Proteinsequenz, des Molekulargewichts und des vorhergesagten offenen Leserahmens wird die VP1-kodierende Sequenz mittels PCR amplifiziert. Als Template wird virale DNA verwendet, die durch Phenol-Chloroform-Extraktion und anschließende Äthanol-Präzipitation aus HaPV-Virionen gewonnen wird. Für die PCR werden 2 Oligonukleotidprimer verwendet, die das Initiationskodon (ATG) bzw. Terminationskodon (TAA; komplementäre Sequenz) und Spaltorte für die Restriktionsendonuklease XbaI am 5'- und 3'-Ende enthalten: VP1-Xba-N2: 5′-A CGT CTA GAT ATG GCC CCA AAA AGA AAA AGC GGC-3′ VP1-Xba-C: 5'-TCT AGA TTA GTT TGC TGG TTT TGC AGG GGG-3' Das erhaltene PCR-Amplifikat wird mit XbaI gespalten und in einen *E. coli*-Vektor (pFR36) inseriert. Rekombinante Plasmide werden durch Restriktionsendonuklease-Verdau und DNA-Sequenz-Analyse charakterisiert.

### 2. Herstellung von Hefe-Expressionsvektoren

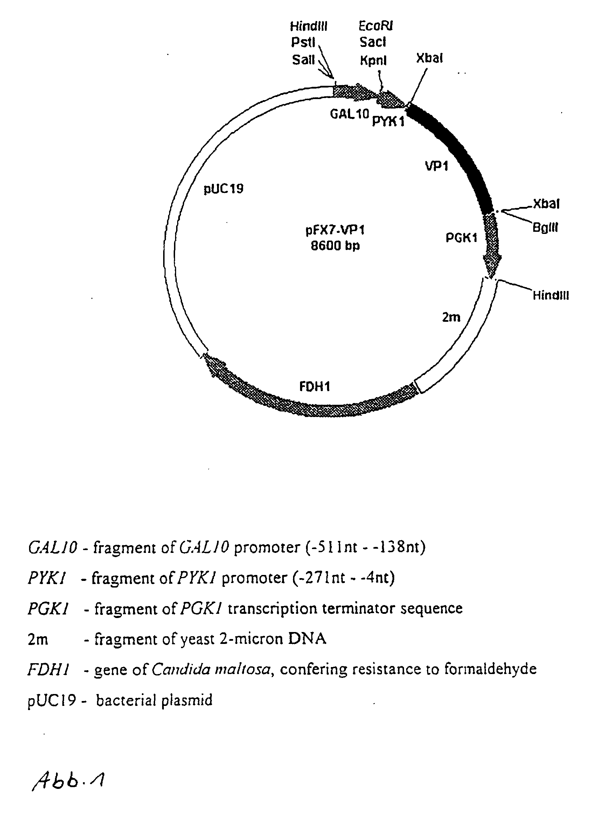

Das Hefeexpressionsplasmid, vorzugsweise pFX7 (Abb. 1), enthält
- einen Hefe-spezifischen Promoter (GAL10-PYK1-Hybrid-Promoter, der aus einem -138 bis - 511 Nukleotid-Fragment von GAL10 mit GAL1-10 UAS-Sequenzen und der -4 bis -271-Sequenz von PYK1 besteht),
- eine Transkriptionsterminationssequenz (von PGK1),
- ein 2 µm DNA-Fragment und
- einen dominanten Selektionsmarker (FDH1-Gen von *Candida maltosa*)
- und einen unikalen Restriktionsort, zwischen Promoter und Terminator, für die Insertion von Fremdsequenzen (XbaI).

Das VP1-kodierende Segment (mit eigenem Translationsinitiations- und - terminationssignal) wird mit XbaI aus pFR36-VPl herausgeschnitten und in den mit XbaI linearisierten Hefeexpressionsvektor pFX7 ligiert. Rekombinante Plasmide werden nach Transformation in *E*. *coli* K12 (XL-1 Blue) selektioniert und durch Restriktionskartierung charakterisiert.

### 3. Expression des Hauptkapsidproteins vom Polyomavirus in Hefe

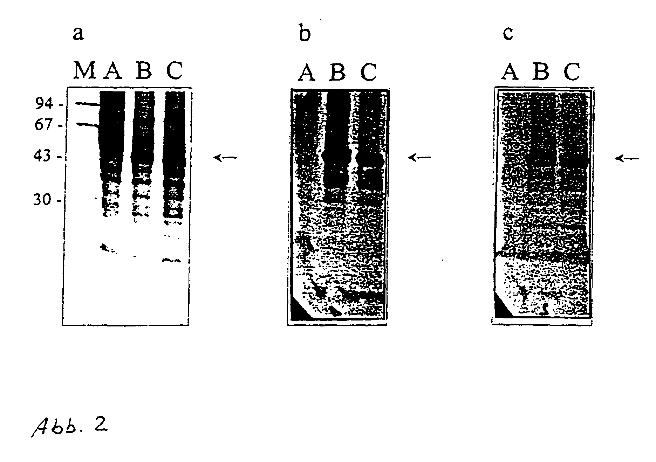

Das HaPV-VP1-Expressionsplasmid wird in Hefezellen, vorzugsweise in *Saccharomyces cerevisiae,* Stamm AH22 (leu2 his4) transformiert. Die Anzucht der transformierten Hefezellen erfolgt in YEPD-Medium (1% Hefeextrakt, 2% Pepton, 2% Glukose) mit 5 mM Formaldehyd. Nach 18stündiger Anzucht bei 30 °C werden die Zellen geerntet, in YEPG-Medium (1% Hefeextrakt, 2% Pepton, 3% Galaktose) mit Formaldehyd re-inokuliert und weitere 24 Stunden kultiviert. Für die Expressionskontrolle wird ein Aliquot entnommen und mit Proben-Puffer denaturiert. Nach Auftrennung im SDS-Polyacrylamidgel wird das rekombinante HaPV-VP1 im Western blot unter Verwendung eines VP1-spezifischen Kaninchenserums nachgewiesen (Abb. 2).

### 4. Reinigung von virus-ähnlichen Partikeln

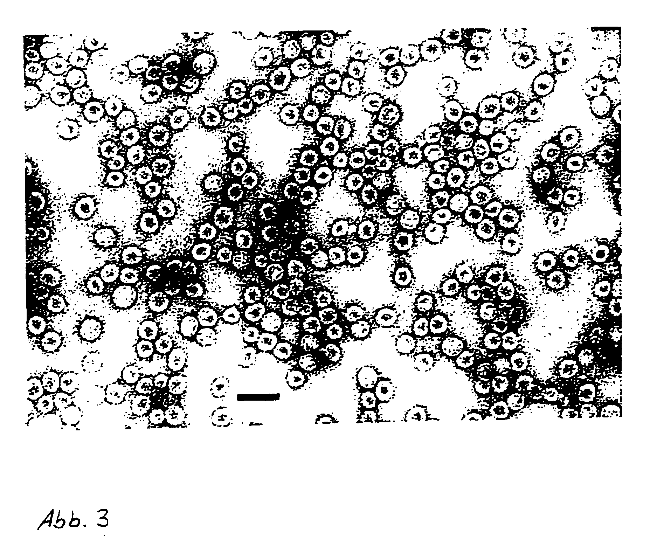

Die, wie unter 3. beschrieben, angezüchteten Hefezellen werden nach Sedimentation in 1 ml Lysepuffer (20 mM Natriumphosphat, pH 7,2; 100 mM NaCl; 2 mM PMSF) resuspendiert und nach Zugabe von 1 g Glasperlen (0,5 mm) durch Vortexing für 4 Minuten bei 4 °C aufgeschlossen. Die entstandenen Lysate werden mit dem doppelten Volumen Lysepuffer verdünnt und 10 Minuten bei 5000 g zentrifugiert. 8,5 ml einer 45%igen Saccharoselösung (w/v; in Lysepuffer) werden mit 1,5 ml des Überstandes überschichtet. Die Partikel werden durch 4stündige Zentrifugation bei 100 000 g im Beckman Ti70.1-Rotor sedimentiert. Das Sediment wird in Lysepuffer (20 mM Natriumphosphat, pH 7,2: 100 mM NaCl und 2 mM PMSF) resuspendiert. Mit dieser Suspension wird ein vorher geschichteter Cäsiumchloridgradient (1,38, 1,35, 1,32, 1,29, 1,26 und 1,23 g/ml in 50 mM Tris-HCl, pH 7,5) beladen und 15 Stunden bei 100 000 g im Beckman Ti70.1-Rotor zentrifugiert. Fraktionen werden gesammelt und mittels SDS-Polyacylamid-Gelelektrophorese und Western blot analysiert. Die Bildung von HaPV-VP1-Partikeln wird durch negative staining mit 1% Uranylacetat und elektronenmikroskopische Analyse bewiesen (Abb. 3). Partikel-enthaltende Fraktionen werden gegen Lysepuffer dialysiert.

### Abb. 1

Schematische Darstellung des Hefe-Expressionsplasmids pFX7-VP1, das die Hamsterpolyomavirus (HaPV)-VP1-kodierende Sequenz enthält.

### Abb. 2

Nachweis der Expression von HaPV-VP1 in Hefezellen.
Totallysate von Hefezellen wurden im 15%igen SDS-Polyacrylamidgel aufgetrennt, mit Coomassieblau-gefärbt (a)
bzw. auf Nitrocellulose transferiert und mit Kaninchen anti-HaPV-VP1 Serum (b) bzw. einem Serum eines HaPV-infizierten Hamsters (c).
Bahnen B und C: HaPV-VP1-exprimierende Hefezellen
Bahn A: Hefezellen ohne VP1-Expressionsplasmid
Bahn M: Molekulargewichtsmarkerproteine
Die VP1-Proteinbande ist durch Pfeile markiert.

### Abb. 3

Nachweis von HaPV-VP1-abgeleiteten virusähnlichen Partikeln aus Hefezellen.
Elektronenmikroskopische Analyse nach Negativkontrastierung.
Vergrößerung: 100 000fach.

## Patentansprüche

1. Verfahren zur Gewinnung von virusähnlichen Partikeln, **gekennzeichnet durch** die Expression des Hauptkapsidproteins VP1 von Polyomaviren in Hefezellen.

2. Verfahren nach Anspruch 1, **dadurch gekennzeichnet, daß** als Hefezellen *Saccharomyces cerevisiae,* Stamm AH22 (leu2, his4) verwendet werden.

3. Verfahren nach Anspruch 1 bis 2, **dadurch gekennzeichnet, daß** das VP1 des Hamsterpolyomavirus (HaPV) verwendet wird.

4. Verfahren nach Anspruch 1 bis 3, **dadurch gekennzeichnet, daß** als Hefe-spezifischer Promoter ein GAL10-PYK1-Hybrid-Promoter verwendet wird.

5. verfahren nach Anspruch 1 bis 4, **dadurch gekennzeichnet, daß** das Hefeexpressionsplasmid das FDH1-Gen von *Candida maltosa* als dominanten Selektionsmarker trägt.

6. Verfahren nach Anspruch 1 bis 5, **dadurch gekennzeichnet, daß** die Hefezellen in YEPD-Medium (1% Hefeextrakt, 2% Pepton, 2% Glukose) mit 5 mM Formaldehyd angezüchtet werden und anschließend in YEPG-Medium (1% Hefeextrakt, 2% Pepton, 3% Galaktose + Formaldehyd) induziert werden.

7. Verfahren nach Anspruch 1 bis 6, **dadurch gekennzeichnet, daß** die Hefezellen durch Glasperlen aufgeschlossen werden.

8. Verfahren nach Anspruch 1 bis 7, **dadurch gekennzeichnet, daß** die virusähnlichen Partikel durch Ultrazentrifugation sedimentiert werden.

9. Verfahren nach Anspruch 1 bis 8, **dadurch gekennzeichnet, daß** die virusähnlichen Partikel durch Ultrazentrifugation im Cäsiumchloridgradienten gereinigt werden.

## Claims

1. Method for obtaining virus-like particles, wherein there is the expression of the main capside protein VP1 of polyomaviruses in yeast cells.

2. Method according to Claim 1, wherein *Saccharomyces cerevisiae,* strain AH22 (leu2, his4) are used as yeast cells.

3. Method according to Claims 1 to 2, wherein the VP1 of the hamster polyomavirus (HaPV) is used.

4. Method according to Claims 1 to 3, wherein a GAL10-PYK1 hybrid promoter is used as a yeast-specific promoter.

5. Method according to Claims 1 to 4, wherein the yeast expression plasmid bears the FDH1 gene of *Candida maltosa* as the dominant selection marker.

6. Method according to Claims 1 to 5, wherein the yeast cells are bred in a YEPD medium (1% yeast extract, 2% peptone, 2% glucose) with 5 mM of formaldehyde and are then induced in YEPG medium (1% yeast extract, 2% peptone, 3% galactose + formaldehyde).

7. Method according to Claims 1 to 6, wherein the yeast cells are solubilised by glass pearls.

8. Method according to Claims 1 to 7, wherein the virus-like particles are sedimented by ultra-centrifugation.

9. Method according to Claims 1 to 8, wherein the virus-like particles are cleaned by ultra-centrifugation in the caesium chloride gradient.

## Revendications

1. Procédé de fabrication de particules similaires à un virus, **se caractérisant par** l'expression de la protéine capsidique principale VP1 du polyomavirus dans des levures.

2. Procédé selon la revendication 1, **se caractérisant par le fait que** l'on utilise le *saccharomyces cerevisiae,* souche AH22 (leu2, his4) en tant que levure.

3. Procédé selon la revendication 1 à 2, **se caractérisant par le fait que** l'on utilise le VP1 du polyomavirus du hamster (HaPV).

4. Procédé selon la revendication 1 à 3, **se caractérisant par le fait que** l'on utilise un promoteur hybride GAL10-PYK1 en tant que promoteur spécifique à la levure.

5. Procédé selon la revendication 1 à 4, **se caractérisant par le fait que** le plasmide de l'expression de la levure porte le gène FDH1 de *Candida maltosa* en tant que marqueur de sélection dominant.

6. Procédé selon la revendication 1 à 5, **se caractérisant par le fait que** les levures sont cultivées dans un milieu YEPD (1 % d'extrait de levure, 2 % de peptone, 2 % de glucose) avec 5 mM formaldéhyde et ensuite induites dans un milieu YEPG (1 % d'extrait de levure, 2 % de peptone, 3 % de galactose + formaldéhyde).

7. Procédé selon la revendication 1 à 6, **se caractérisant par le fait que** les levures sont ouvertes par des perles de verre.

8. Procédé selon la revendication 1 à 7, **se caractérisant par le fait que** les particules similaires à un virus sont sédimentées par ultracentrifugation.

9. Procédé selon la revendication 1 à 8, **se caractérisant par le fait que** les particules similaires à un virus sont nettoyées par ultracentrifugation dans le gradient du chlorure de césium.
